# EUROPEAN PATENT SPECIFICATION

(11) **EP 1 337 260 B1**
(45) Date of publication and mention of the grant of the patent: **16.03.2005**
(21) Application number: 00981644.8
(22) Date of filing: 22.11.2000
(51) Int. Cl.: A61K 31/715, A61K 9/00, A61K 47/02, A61K 47/36

(54) **PREPARATION FOR STRENGTHENING THE IMMUNE RESPONSE IN THE WALDEYER'S RING AND THE ORONASAL MUCOSA BY INTRANASAL ADMINISTRATION**
ARZNEIZUBEREITUNG ZUR INTRANASALEN VERABREICHUNG UM DIE IMMUNANTWORT IN DER MUND- UND NASENSCHLEIMHAUT UND IM WALDEYER-RING ZU VERSTÄRKEN
PREPARATION POUR LE RENFORCEMENT DE LA REPONSE IMMUNITAIRE DANS L'ANNEAU LYMPHATIQUE DE WALDEYER ET LA MUQUEUSE BUCCO-NASALE POUR ADMINISTRATION INTRANASALE

(43) Date of publication of application: 27.08.2003
(73) Proprietor: D.M.G. Italia Srl, 00181 Roma (IT)
(72) Inventor: MERCURI, Luigi, I-00181 Roma (IT)
(74) Representative: Sarpi, Maurizio
(86) International application number: PCT/IT2000/000479
(87) International publication number: WO 2002/041900

(56) References cited:
- EP-A- 0 045 718
- WO-A-87/01037
- FR-A- 2 780 285
- US-A- 5 519 009

## Description

The present invention relates to a product to be administered by nose for preventing inflammatory or hypertrophic forms and strengthening the immune action in the Waldeyer's ring and the oronasal mucosa.

Such product consists of a physiological solution with a high concentration in a polysaccharide such as glucan.

In particular, two solutions having different concentrations have been specified:
- a first physiological solution with a glucan concentration equal to 0.25% for cleaning the nasal fossae and with a preventive hydrating, reepithelializing capability of strengthening the immune action in the Waldeyer's ring and the oronasal mucosa, and
- a physiological solution with a glucan concentration equal to 2% for hydrating and reepithelializing the nasal mucosa, capable of strengthening the immune response in the Waldeyer's ring during the acute phase of the inflammatory, hypertrophic forms of the same.

The inventive concept upon which the invention is based provides the stimulation of the immune system of the tonsillary Waldeyer's ring and the oronasal mucosa by intranasal administration of a polysaccharide capable of activating the tissular macrophages.

It should be remembered that the Waldeyer's ring is the lymphatic tissue located around the inlet of nasopharynx and oropharynx like a ring. It is formed of the pharyngeal, palatal, lingual tonsillae and has the function of blocking microorganisms and exogenic substances at such inlet.

Considering the very high absorption capability of the nasal mucosae and the large amount of immune material in such mucosae and the lymphoid tissue of the Waldeyer's ring annexed thereto, it is believed according to the invention that repeated contacts with a polysaccharide solution having immunotrophic activity can prevent the inflammatory or hypertrophic forms thereof or even contribute to their improvement. It is widely known that the monocytes are mononucleate cells of blood and take part in our defence mechanism. Upon migration to the tissues, they maturate, grow, and acquire the capability of phagocytizing. Once activated, tissular macrophages or histiocytes begin to secrete IL-1 (interleukin 1), a proteic messenger that is able to catalyze t-helper lymphocytes by inhibiting a particular receptive site thereof. The active macrophages in the tissues are capable of providing a good defence mechanism as they have a large aspecific phagocytizing ability.

It is also known that tissular macrophages or histiocytes can be activated, by the presence of an invader that can also be identified by a polysaccharide chain capable of inhibiting the receptive macrophage site CR3 by the stimulation of the properdin, a serumal protein able to activate the complement complex, and by concretizing a reaction product composed of B and D factors of such complex. Among many polysaccharides of biological importance, the attention is drawn to β-(1-3)-glucan which is a polysaccharide molecule extracted from the cellular wall of *Saccharomyces cerevisiae.*

### β- (1-3) -glucan has two extremely significant characteristics, namely:

a) the activity of such molecule as biological modifier of the immune response of derma is widely proved. β-(1-3)-glucan has been identified as a strong stimulator of the immunosystem with the capability of activating macrophages, neutrophils and other cells carrying specific β-glucan receptors on their surface. The activation of such cells by glucan stimulates the non-specific defence mechanism of the host. Other polysaccharides such as mannans, galactans, and polymers of glucose chains α(1-4) or β(1-4) haven't got such activity;
b) recent studies have also pointed out the glucan absorption capability of the mucosae by intranasal administration ("Protective Immunity against *Streptococcus mutans* Infection in Mice after Intranasal Immunization with the Glucan-Binding Region of S. mutans Glucosyltransferase", Infection and Immunity, Dec. 1999, p. 6543-6549, Vol. 67, No. 12). According to the invention, therefore, it is provided a contact absorption of a proportion of β-(1-3)-glucan by intranasal administration in order to prevent the inflammatory or hypertrophic forms of the Waldeyer's ring and/or to contribute to their improvement also by the mechanical action performed by the physiological solution.

Therefore, the object of the present invention is a water saline isotonic solution, particularly an isotonic solution of sodium chloride (0.9%) typically used for the parenteral administration of substances in which β- (1-3) -glucan is dissolved.

Following the clinical experimentation carried out, two different glucan concentrations capable of providing specific therapeutic responses have been identified, namely:
1) a 0.9% Na-solution (physiological solution) with a glucan concentration equal to 0.25% that can be packed in phials of several types and is able to clean the nasal fossae with a preventive capability of strengthening the immune action in the Waldeyer's ring;
2) a 0.9% Na-solution (physiological solution) with a glucan concentration equal to 2% packed in phials of several types and able to hydrate the nasal mucosa with a capability of strengthening the immune action in the Waldeyer's ring during the acute phase of the inflammatory forms of the same and its hypertrophic forms.

A very delicate preservative such as sodium benzoate or potassium sorbate may be added to the solutions mentioned above to an adequate extent.

As the proximate principle of the disclosed product is only β- (1-3) -glucan considered as an alimentary component of human diets, the disclosed product may be indifferently used both in adults and children.

With regard to this, in order to facilitate the use there are provided confections of single-dose phials with different capacities that can be closed, for example 3.5 or 10 ml.

## Claims

1. Use of polysaccharide for the preparation of a medicament, under the form of solution to be administered by nose, to prevent inflammation and hypertrophy in the Waldeyer's ring and the oronasal mucosa.

2. Use of polysaccharide according to claim 1 **characterized in that** the polysaccharide is glucan.

3. Use of polysaccharide according to claim 1 **characterized in that** in the medicament glucan is in a concentration between 0.1 and 4 % in weight.

4. Use of polysaccharide according to claim 1 **characterized in that** the medicament comprises 9% isotonic solution of sodium chloride and glucan in a concentration equal to 0.25% by weight.

5. Use of polysaccharide according to claim 1 **characterized in that** the medicament comprises 0.9% Na-solution (physiological solution) and glucan in a concentration equal to 2% by weight.

6. Use of polysaccharide according to claim 2 **characterized in that** glucan is in the form of β- (1-3)-glucan.

7. Use of polysaccharide according to claim 1 **characterized in that** the medicament is packed in single-dose phials.

## Patentansprüche

1. Verwendung von Polysaccharid zur Herstellung eines Medikaments in der Form einer Lösung, die über die Nase verabreicht wird, um Entzündung und Hypertrophie im Waldeyerschen Rachenring und der Schleimhaut von Mund und Nase zu verhindern.

2. Verwendung von Polysaccharid gemäß Anspruch 1, **dadurch gekennzeichnet, dass** das Polysaccharid Glucan ist.

3. Verwendung von Polysaccharid gemäß Anspruch 1, **dadurch gekennzeichnet, dass** in dem Medikament Glucan in einer Konzentration zwischen 0,1 und 4 Gewichtsprozent vorliegt.

4. Verwendung des Polysaccharides gemäß Anspruch 1, **dadurch gekennzeichnet, dass** das Medikament eine 9 % isotonische Lösung von Natriumchlorid und Glucan in einer Konzentration gleich 0,25 Gewichtsprozent umfasst.

5. Verwendung des Polysaccharides gemäß Anspruch 1, **dadurch gekennzeichnet, dass** das Medikament eine 0,9 %-ige Na-Lösung (physiologische Lösung) und Glucan in einer Konzentration gleich 2 Gewichtsprozent umfasst.

6. Verwendung des Polysaccharides gemäß Anspruch 2, **dadurch gekennzeichnet, dass** Glucan in der Form von β- (1-3) -Glucan vorliegt.

7. Verwendung des Polysaccharides gemäß Anspruch 1, **dadurch gekennzeichnet, dass** das Medikament in Ampullen für eine Dosis abgepackt ist.

## Revendications

1. Emploi d'un polysaccharide pour la préparation d'un médicament, sous la forme d'une solution à administrer par le nez, pour prévenir l'inflammation et la hypertrophie dans l'anneau de Waldeyer et la muqueuse oronasale.

2. Emploi d'un polysaccharide selon la Revendication 1 **caractérisé en ce que** le polysaccharide est glucan.

3. Emploi d'un polysaccharide selon la Revendication 1 **caractérisé en ce que** le glucan médicament est en concentration parmi 0,1 et 4% en poids.

4. Emploi d'un polysaccharide selon la Revendication 1 **caractérisé en ce que** le médicament comprend 9% d'une solution isotonique de chlorure de sodium et glucan en concentration égale à 0,25% en poids.

5. Emploi d'un polysaccharide selon la Revendication 1 **caractérisé en ce que** le médicament comprend 0,9% de solution de Na (solution physiologique) et glucan en concentration égale à 2% en poids.

6. Emploi d'un polysaccharide selon la Revendication 2 **caractérisé en ce que** du glucan est présent dans la forme de β-(1-3)-glucan.

7. Emploi d'un polysaccharide selon la Revendication 1 **caractérisé en ce que** le médicament est conditionné en ampoules unidose.
